# EUROPÄISCHE PATENTSCHRIFT

(11) **EP 2 214 762 B1**
(45) Veröffentlichungstag und Bekanntmachung des Hinweises auf die Patenterteilung: **13.05.2020**
(21) Anmeldenummer: 08784196.1
(22) Anmeldetag: 06.06.2008
(51) Int. Cl.: A61M 16/06

(54) **ATEMMASKE MIT EINEM FÜLLKÖRPER SOWIE HERSTELLUNGSVERFAHREN**
RESPIRATORY MASK COMPRISING A FILLING BODY AND PRODUCTION METHOD
MASQUE RESPIRATOIRE POURVU D'UN CORPS DE REMPLISSAGE, ET PROCÉDÉ DE FABRICATION CORRESPONDANT

(30) Priorität: 23.11.2007 DE 102007057091
(43) Veröffentlichungstag der Anmeldung: 11.08.2010
(73) Patentinhaber: Löwenstein Medical Technology S.A., 2557 Luxembourg (LU)
(72) Erfinder: EIFLER, Martin, 25348 Glückstadt (DE)
(74) Vertreter: Klickow & Wetzel PartGmbB
(86) Internationale Anmeldenummer: PCT/DE2008/000972
(87) Internationale Veröffentlichungsnummer: WO 2009/065368

(56) Entgegenhaltungen:
- EP-A- 1 982 740
- WO-A-93/00125
- WO-A-99/58198
- WO-A-03/105921
- DE-A1-102007 042 733
- US-A- 5 343 878
- US-A1- 2006 076 018
- US-A1- 2006 185 675

## Beschreibung

Die Erfindung betrifft eine Beatmungsmaske, wie in dem Oberbegriff des Patentanspruchs 1 beschrieben.

In der medizinischen Versorgung werden die Patienten mit verschiedensten Atemstörungen durch Beatmungssysteme mit Luft, bzw. mit Sauerstoff, mit Hilfe von Geräten, die Luft oder ein Luftgemisch als Atemgas zur Nase und/oder zum Mund zuführen, versorgt. Eine wichtige Rolle hierbei spielt die Kontaktschnittstelle zwischen den ein Luftgemisch zuführenden Schläuchen und den Atemöffnungen des Menschen, bzw. eines Lebewesens, da auch Tiere mitunter versorgt werden können.

Diese Schnittstelle ist gewöhnlich als eine Atemmaske ausgeführt, die nur die Nase oder den Mund, oder die Nase und den Mund abdeckt, vorzugsweise mittels flexibler Bänder am Kopf befestigt wird und einen oder zwei Schlauchanschlüsse aufweist. Die Atemmaske muss stundenlang, bzw. nächte und tagelang, auf dem Gesicht eines Patienten verweilen, wodurch hohe Anforderungen an den Tragekomfort gestellt sind. Beschwerden durch Druckstellen an den Gesichtspartien und insbesondere an dem Nasenrücken belasten den Patienten. Zusätzlich kommt hinzu das Problem der Größenanpassung der Masken an verschiedene Gesichts- und Nasenbeschaffenheiten der Patienten.

Um sowohl das Komfortproblem, wie auch das Größenproblem zu lösen, wurden Atemmasken entwickelt, die eine der Gesichtsform angepasste Atemwegeglocke, welche auch als Maskenoberteil oder Maskenwulst bezeichnet werden kann, umfassen, die aus einem flexiblen Material, vorzugsweise einem hautfreundlichen Silikon, einem thermoplastischen Elastomer (TPE) oder aus Polyurethan, hergestellt und federnd ausgeführt ist, und nach innen einen umlaufenden Lippenkontaktkranz aufweist. Das Maskenoberteil dient bestimmungsgemäß der Anlage der Beatmungsmaske am Gesicht des Patienten und bildet somit einen dichtenden Kontakt zwischen der Beatmungsmaske und dem Gesicht aus.

Ein Gelwust aus dem Stand der Technik ist aus einem Gel hergestellt, welches in einer schützenden PU-Folie eingeschlossen ist. Eine zusätzliche Silikonhülle liegt zwischen dem Gesicht des Patienten und dem PU-Gelkörper. Die Wanddicken der PU-Folie und der Silikonhülle sind im Wesentlichen konstant gehalten.

Die WO 2005/094928 A1 beschreibt eine Beatmungsvorrichtung mit einer Atemmaske, in deren Maskenoberteil ein Gelwulst eingelegt ist, der auf die Lippen der Maskenoberteils einwirkt und so mehr Druck auf die Gesichtspartien aufbringt. Hierdurch wird auch eine bessere Abdichtung angestrebt, um die Luftzufuhr genauer und ökonomischer betreiben zu können. Der Gelwulst ist im Wesentlichen gleichmäßig dick gestaltet, hat auch einen andrückenden Teil im Bereich des Nasenrückens und verschlechtert somit die Anpassungsfähigkeit der Maskenoberteils an die Form der Gesichtspartien des Patienten, bzw. macht die Herstellung von individuell für den Patienten geformten Teilen erforderlich, wodurch der Aufwand und mit ihm die Kosten steigen Weitere beispiele sind bekannt durch US2006076018 und DE102007042733.

Derartige bekannte Lösungen haben zur Folge, dass die Anzahl anzubietender und verschieden geformter Maskenoberteile groß ausfallen muss, um verschiedene Gesichtsformen und Größen abzudecken, wenn man bei dem Tragekomfort und der erreichbaren Dichtheit keine Abschläge in Kauf nehmen will.

Daher stellt sich die vorliegende Erfindung zur Aufgabe, die oben geschilderten Probleme zu lösen und ein derartiges Maskenoberteil einer Atemmaske vorzuschlagen, welches den auf die Gesichtspartien ausgeübten Kontaktdruck optimal gestalten lässt, dabei eine größere Anpassungsfähigkeit an verschieden große und verschieden geformte Gesichtspartien der Patienten ermöglicht, um mit einer geringeren Anzahl verschiedener Maskengrößen auszukommen, und hierbei weniger Einzelteile aufweist. Ferner stellt sich die Erfindung die Aufgabe, allgemein den Tragekomfort einer Atemmaske zu verbessern und eine kostengünstige Herstellung zu ermöglichen.

Die gestellte Aufgabe wird im Wesentlichen mit den kennzeichnenden Merkmalen des Patentanspruchs 1 gelöst, wobei das Maskenoberteil einen Hohlraum erhält, der mit einem Füllstoff bestimmter Konsistenz und Elastizität gefüllt wird.

Der Füllstoff kann unterschiedlichste Beschaffenheiten aufweisen, angefangen von den bereits erwähnten Gelmaterialien, wie Polyurethan- oder Silikon-Gel, bzw. das Naturgel Agarose, bis hin zu Schaumstoffen, Gasmischungen und Flüssigkeiten, wie beispielsweise die oft in der Medizin verwendete Kochsalzlösung. Letzten Endes kann es ebenso das gleiche Material sein, aus dem das Maskenoberteil hergestellt ist, insbesondere wenn es aufgeschäumt wird. Durch die erfindungsgemäße integrierte Bauweise lässt sich die Dicke der Wandungen des Maskenoberteils sehr genau den Erfordernissen anpassen, um einigen Teilbereichen des Maskenoberteils genau nach Bedarf mehr oder weniger Steifigkeit zu verleihen.

Die Erfindung kann Anwendung finden bei Nasal- und Vollgesichtsmasken, in der Notfall-, Heim-, und Klinikbeatmung. Das erfindungsgemäße Maskenoberteil ist beispielsweise für die CPAP-, APAP-, Bilevel- und Heimbeatmung geeignet. Das erfindungsgemäße Maskenoberteil ist für einen typischen Druckbereich des Atemgases im Bereich von 0 mbar bis 50 mbar geeignet.

Zur Herstellung des erfindungsgemäßen Maskenoberteils wird vorgeschlagen, das Maskenoberteil mit formgebenden Verfahren aus einem elastischen Kunststoff, vorzugsweise einem Silikon oder Polyurethan, mit mindestens einem Hohlraum herzustellen und anschließend in diesen Hohlraum den vorgesehenen Füllstoff einzufüllen. Die Befüllung soll vorzugsweise durch mindestens eine für diesen Zweck vorgesehene Öffnung erfolgen, die vorzugsweise nicht in den Bereichen des Maskenoberteils angebracht ist, die in Kontakt mit der Gesichtshaut kommen.

Die Befüllung erfolgt besonders bevorzugt durch Injizieren des Füllmaterials, wobei die Hülle vorzugsweise in den dickeren Bereichen des Maskenoberteils durchstochen wird. Die mindestens eine Öffnung wird insbesondere bei der Befüllung mit flüssigen oder gasförmigen Füllstoffen nach der Befüllung, beispielsweise mit einem Stopfen, durch Verschweißung oder durch Verklebung, dicht verschlossen. Insbesondere gasförmige Füllstoffe können hierbei unter Druck gesetzt werden, so dass die Druckstärke die Eigenschaften des Maskenoberteils beeinflussen und bestimmen kann.

Die Gestalt, Größe und örtliche Ausdehnung der befüllten Hohlräume wird erfindungsgemäß so gewählt, dass sich die günstigsten Eigenschaften des Maskenoberteils einstellen. Beispielsweise ist der gefüllte Hohlraum in Form einer Wulst in der Wandung des Maskenoberteils so verteilt, dass entweder fast die gesamte Wandung davon erfasst wird, oder nur spezielle Teilbereiche davon. Vorzugsweise ist der Eckenbereich des Maskenoberteils mit einer örtlichen aufgefüllten Wulst zu versehen, um diesem Bereich eine gewünschte Eigenschaftenkombination aus Elastizität und Steifigkeit zu verleihen.

Ferner wird in weiterführenden Ausführungen der vorliegenden Erfindung vorgeschlagen, die Eigenschaften des Füllstoffs je nach Befüllungsort innerhalb des erfindungsgemäßen Hohlraums des Maskenoberteils zu variieren, um hierdurch zusätzlich oder alternativ zur erfindungsgemäß variierten Dicke des Hohlraums durch eine ortsabhängige Elastizität und Viskosität des Füllstoffs eine noch bessere Anpassung der resultierenden, den Tragekomfort fördernden Eigenschaften des Maskenoberteils an die Erfordernisse eines Einsatzes auf dem Gesicht eines Patienten zu erreichen. Die Kombination verschiedener Füllstoffe gehört ebenso zu erfindungsgemäßen Ausführungen der vorliegenden Erfindung.

Ein alternatives Herstellverfahren ergibt sich dadurch, dass man zunächst einen aus einem vorgesehenen Füllstoff geformten Füllkörper herstellt und diesen anschließend mit einer schützenden Hülle in einem Tauchverfahren umhüllt oder in einer Form umspritzt. Bei der Verwendung von gasförmigen oder flüssigförmigen Füllstoffen kann es ein geformter Beutel sein, der zu befüllen und mit einer schützenden Schicht zu überziehen ist.

Erfindungsgemäß wird ferner vorgeschlagen, im Bereich des Nasenrückens auf eine Unterlegung der lippennahen Bereiche des Maskenobeteils mit einem Füllstoff ganz zu verzichten, bzw. den zu befüllenden Hohlraum sehr dünn zu gestalten, um keinen zu hohen Druck auf den empfindlichen Nasenrücken zu erzeugen. Die Luftdichtheit in dem Nasenrückenbereich wird vorzugsweise allein mit Hilfe der nach innen geformten Kontaktlippen erreicht, die durch ihre hohe Flexibilität auch eine gewisse größere Anpassungsfähigkeit an verschieden große Nasen der Patienten ermöglichen.

Ferner wird vorgeschlagen, einen Füllstoff, wie Gel oder Schaumstoff, nicht in einen Hohlraum in das Maskenoberteil einzubringen, sondern einfach auf die Innenseite oder die Außenseite der Wandungen des Maskenoberteils aufzubringen. Diese Lösung bietet ökonomische Vorteile, da die bestehenden Formwerkzeuge für Maskenoberteile weiter verwendet werden. Um die so auf die Wandungen der Maskenoberteile aufgebrachten Füllkörper gegen eine Verschmutzung, bzw. einen Hautkontakt zu schützen, werden deren offene Oberflächen anschließend mit einer schützenden Schicht versehen. Die schützende Schicht ist entweder aus dem angeschmolzenen Füllstoffmaterial oder aus einem dritten aufzubringenden Stoff hergestellt. Die Form und die erfindungsgemäße variierende Dicke des mindestens einen so aufgebrachten, vorzugsweise aufgespritzten oder aufgeschäumten wulstförmigen Füllkörpers wird durch ein Verfahren umgesetzt, bei dem aus einer Düse der Füllstoff, beispielsweise pulverisiert, durch verschiedene Mengen oder unterschiedliche Dosierung oder verschieden lange Verweilzeiten, geformt wird.

Die vorliegende Erfindung ermöglicht eine verbesserte Anpassung der durch das Maskenoberteil der Atemmaske auf die Gesichtspartien ausgeübten Druckkräfte und eine für den praktischen Gebrauch vorteilhafte einteilige Ausführung des Maskenoberteils.

Es wird darauf hingewiesen, dass der Umfang der vorliegenden Erfindung nicht allein auf die vorgestellten bevorzugten Ausführungen eingeschränkt ist und weitergehende Kombinationen und Varianten der vorliegenden Erfindung einem durchschnittlichen Fachmann aus den Haupt und Unteransprüchen erschließbar sind, ohne dass hierfür die Lehre der vorliegenden Erfindung verlassen werden müsste.

Ausführungen der vorliegenden Erfindung werden nun anhand von lediglich Beispielen mit Bezug zu den zugehörigen Zeichnungen beschrieben, wobei funktionell gleiche Teile mit entsprechenden Bezugskennziffern bezeichnet sind.
Fig.1 zeigt einen Teilausschnitt durch eine Ausführung des Maskenoberteils mit einem Hohlraum und einem Füllstoff darin,
Fig. 2 zeigt eine Ausführung des Maskenoberteils mit einem begrenzt ausgedehnten Hohlraum,
Fig. 3 zeigt eine Ausführung des Maskenoberteils mit einem begrenzt ausgedehnten Hohlraum im inneren Eckenbereich,
Fig. 4 zeigt eine Ausführung des Maskenoberteils mit einem über die gesamte Innenseite des Maskenoberteils ausgedehnten Hohlraum,
Fig. 5 zeigt eine Draufsicht auf ein Maskenoberteil,
Fig. 6 zeigt eine Gesamtansicht einer Atemmaske,
Fig. 7 zeigt mehrere Querschnitte durch den, mit Füllstoff (15) befüllten, Hohlraum,
Fig. 8 zeigt einen schematischen Längsschnitt durch das Maskenoberteil (3) gemäß Fig. 6 und Querschnitte durch den gefüllten Hohlraum (15) an unterschiedlichen Stellen und
Fig. 9 zeigt eine Ausführung des Maskenoberteils (3) mit bereichsweise unterschiedlichen Wandungsstärken.

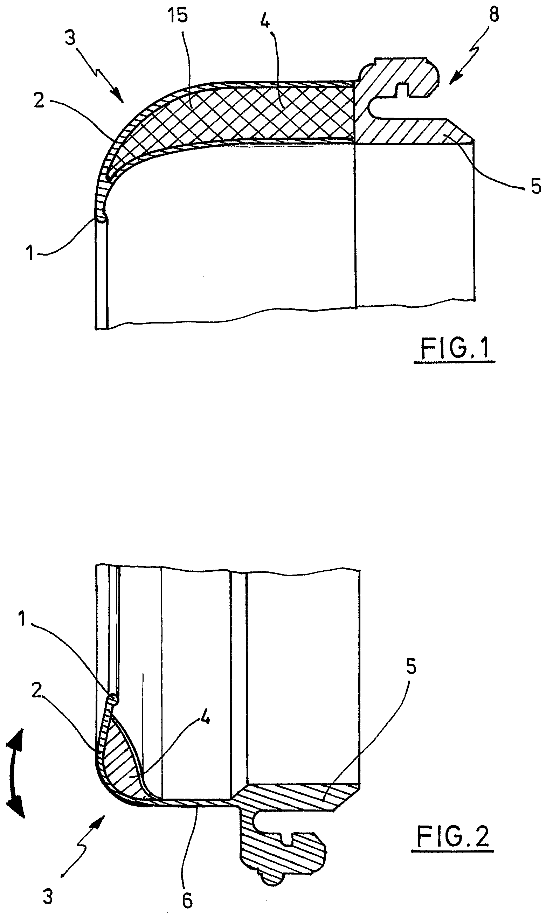

In Figur 1 ist in einem Teilausschnitt ein Maskenoberteil (3) einer Atemmaske (8) zu sehen, in dessen Wandungen ein Hohlraum (4) mit einem darin eingefüllten Füllstoff (15) so gestaltet ist, dass die Dicke, bzw. Tiefe des Füllstoffes (15) im Verlauf des Querschnitts des Maskenoberteiles (3) variiert. Vorzugsweise ist der Patientenkontakt-Bereich des Maskenoberteiles (3) weicher ausgeführt.

Das Maskenoberteil (3) endet auf seiner für die Berührung mit den Gesichtspartien des Patienten vorgesehenen Kontaktseite in einer dünner werdenden Kontaktlippe (1), welche randumschließend das Maskenoberteil (3) mit einer weichen und flexiblen Kontaktzone versieht. Die Wandungen des Maskenoberteils (3) werden mit einem Maskenanschlußkörper (5) abgeschlossen, der entweder aus dem gleichem Material einteilig mit dem Maskenoberteil (3) oder als ein per Klebe- oder Schweißverbindung anzubringendes Teil ausgeführt ist.

Der Maskenanschlußkörper (5) wird mit einem nicht dargestellten Maskenunterteil mechanisch mittels einrastender Teile verbunden, bzw. kann alternativ per Klebe- oder Schweißverbindung unlösbar mit dem Maskenunterteil verbunden werden. Der Hohlraum (4) ist mit einer Außenhaut (2) begrenzt.

Die Dicke, bzw. Tiefe des Füllstoffes (15) können erfindungsgemäß im Verlauf des Querschnitts des Maskenoberteiles (3) auch im wesentlichen konstant sein.

Zur Befüllung des Hohlraums (4) mit einem Füllstoff (15) weist das Maskenoberteil (3) mindestens eine Öffnung auf, die in Fig. 1 nicht dargestellt ist. Diese Öffnung kann an beliebiger Stelle in der Wandung des Maskenoberteils (3) positioniert werden, außer im Bereich der Außenflächen der Kontaktlippen (1) und deren Umgebung, damit sie dort nicht störend auf die zu kontaktierende Gesichtshaut wirken kann. Die Befüllungsöffnung wird vorzugsweise verklebt, verschweißt oder mit einem Stopfen verschlossen.

Figur 2 zeigt eine weitere bevorzugte Ausführung der vorliegenden Erfindung, bei der das Maskenoberteil (3) zumindest bereichsweise einen auf seinen inneren Eckenbereich begrenzten Hohlraum (4) aufweist. Hierdurch lassen sich andere Eigenschaften für das Maskenoberteil (3) vorgeben und ebenso Kosten einsparen. Speziell die Kontaktlippen (1) erhalten eine Druck erzeugende Unterlegung mit einem gewählten Füllstoff, während der Wandbereich (6) frei von einem Füllstoff bleibt.

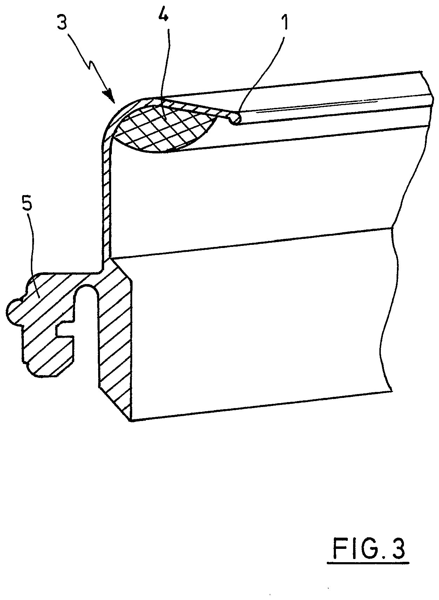

Figur 3 zeigt eine bevorzugte Ausführung mit einem, wie in Fig. 2 auf den Eckenbereich begrenzten, Hohlraum (4), dessen Form speziell nach außen konkav gewölbt ist, wodurch die Übergänge zu den füllstofffreien Wandpartien des Maskenoberteils (3) abrupter gestaltet sind und hierdurch andere, für einige Anwendungen vorteilhafte, Eigenschaften vorgebbar sind.

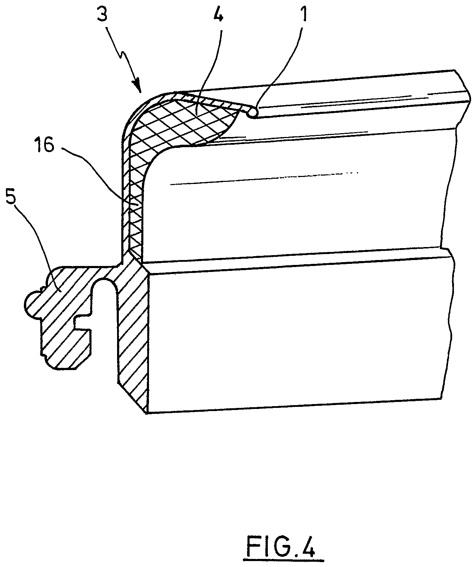

Figur 4 zeigt eine weitere bevorzugte Ausführung der vorliegenden Erfindung, die eine zusätzliche federnde Pufferzone (16) aufweist und hierzu mit einem Hohlraum (4) versehen ist, der sich auch über die zusätzliche federnde Pufferzone (16) erstreckt. Hierdurch ist es möglich, andere vorteilhafte federnden Eigenschaften des Maskenoberteils vorzugeben, die entweder experimentell oder rechnerisch bestimmt werden können.

Durch eine dünne Ausführung der Füllung des Hohlraumes (4) wird beispielsweise eine weiche Pufferzone realisiert. Durch eine Füllung des Hohlraumes (4) mit relativ unflexiblem Material, beispielsweise mit Gel einer Härte über 15 Shore 00, erhält die Pufferzone (16) eine stützende Funktion. Die federnde Pufferzone (16) kann auch derart ausgeführt sein, dass die Füllung zumindest bereichsweise zwei unterschiedliche elastische Eigenschaften aufweist.

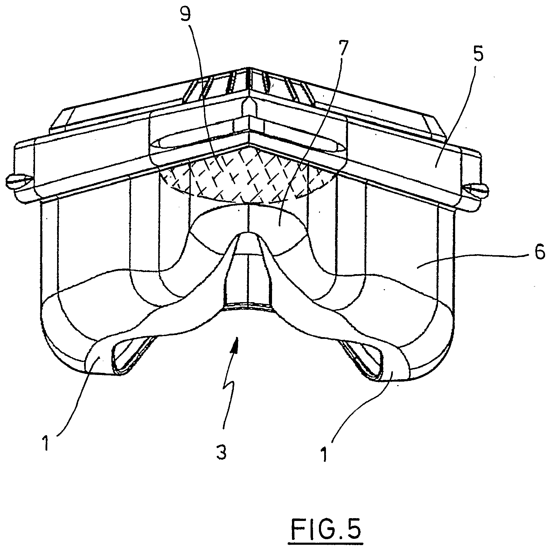

Figur 5 zeigt eine räumliche Darstellung eines Maskenoberteils (3), das über Kontaktlippen (1) verfügt, welche randumschließend und nach innen in das Maskenoberteil hinein geformt gestaltet sind. Die Wandung (6) des Maskenoberteils (3) dient hierbei der Verbindung mit dem nicht dargestellten Maskenkörpers mittels mechanisch einrastender Elemente, und sie enthält mindestens einen Hohlraum mit einem Füllkörper darin, die im Bild nicht dargestellt sind. Der schraffierte Bereich (9) im Nasenrückenbereich (7) des Maskenoberteils ist ohne einen Füllkörper, beispielsweise ohne Gel, ausgeführt. In einer weiteren Ausführungsform der Erfindung ist im gesamten Nasenrückenbereich kein Füllkörper vorgesehen.

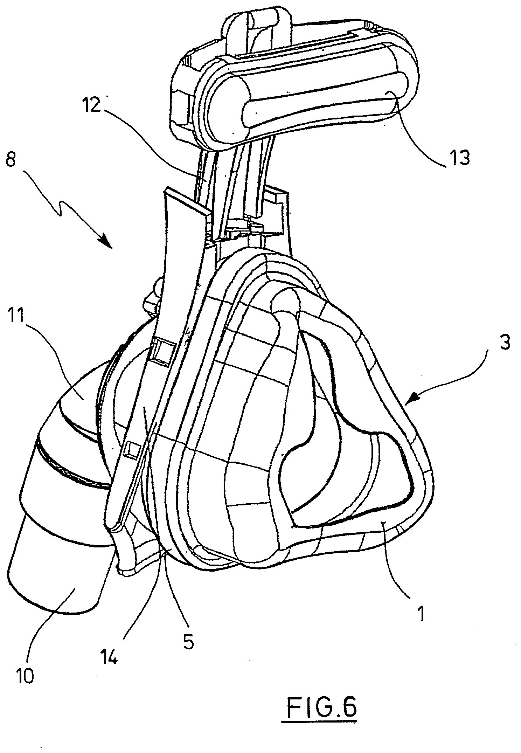

Figur 6 zeigt eine Ausführung der Atemmaske (8) mit dem erfindungsgemäßen Maskenoberteil (3). Das Maskenoberteil(3) ist hierbei mit seinem Maskenanschlussbereich (5) mit dem Maskenkörper (14) mittels einrastender mechanischer Elemente verbunden und die Atemmaske (8) hat ansonsten ihre bekannten Ausstattungsmerkmale, wie eine Drehhülse (10) zum Anschluss eines Luft zuführenden Schlauchs, der mit einem Winkel (11) drehbar mit dem Maskenkörper (14) verbunden ist. An einer Halterung (12) der Stirnstütze ist verschiebbar ein Stirnpolster (13) befestigt, mit dem die Maske zusätzlich einen Halt an der Stirn des Patienten findet. Die Maske wird mit nicht dargestellten Gurten an dem Kopf des Patienten befestigt.

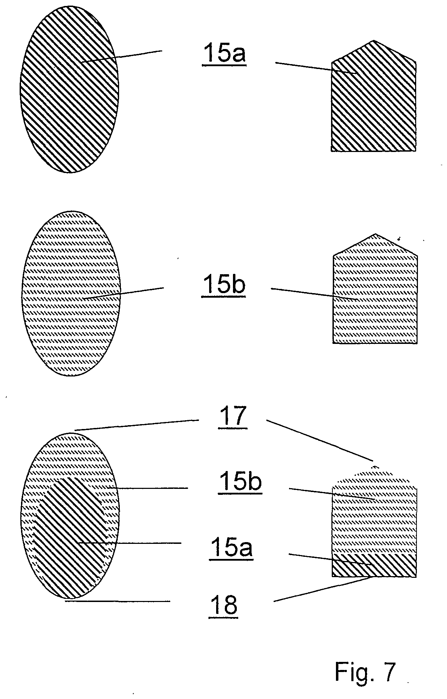

Figur 7 zeigt Querschnitte durch mit Füllstoff (15) befüllte Hohlräume. In Figur 7a und 7b ist der Hohlraum homogen mit einem Füllstoff gefüllt. In Figur 7a ist der Füllstoff (15a) von größerer Härte und von geringerer Elastizität als der Füllstoff in Figur 7b. In Figur 7b ist der Füllstoff (15b) von geringerer Härte und von größerer Elastizität als der Füllstoff in Figur 7a.

In Figur 7c ist der Hohlraum (4) inhomogen mit Füllstoff (15a und 15b) befüllt. Der gesichtsnahe Bereich (17) ist mit dem weicheren Füllstoff (15b) befüllt. Der gesichtsferne Bereich (18) ist mit dem härteren Füllstoff (15b) befüllt. Bevorzugt werden Füllstoffe einer Härte im Bereich 5 Shore 000 bis 15 Shore 00 als weiche Füllstoffe verwendet und Füllstoffe einer Härte > 15 Shore 00 als harte Füllstoffe.

Figur 7 zeigt außerdem, dass der, mit Füllstoff (15) befüllten Hohlraum unterschiedlich geformt sein kann.

Der erfindungsgemäße Füllstoff (15) ermöglicht es, innerhalb eines sehr großen Gestaltungsbereiches, das Maskenoberteil (3) genau in denjenigen Bereichen mit einer vorgebbaren gesteigerten Stabilität zu versehen, wo dies erforderlich ist, und andere Bereiche des Maskenoberteiles (3) mit einer dünnen Wandstärke und/oder sehr weichen Materialkonsistenz zu belassen.

Die vorstehend mehrfach beschriebene Einfüllöffnung kann als eine Öffnung im klassischen Sinne ausgebildet sein, die nach einem Applizieren des Füllstoffes (15) wieder geschlossen wird, insbesondere ist aber daran gedacht, den Füllstoff (15) in einer strömungsfähigen Konsistenz durch ein Injizieren in den Hohlraum (4) einzubringen. Erfolgt dieses Injizieren in einem dickwandigen Bereich in einer Umhüllung des Hohlraumes (4), so schließt sich der Injektionskanal nach einem Zurückziehen des Injektionsmittels selbsttätig aufgrund der elastischen Materialeigenschaften. Die Fertigung wird hierdurch wesentlich erleichtert.

Gemäß der vorstehenden Beschreibung besteht eine bevorzugte Ausführungsform darin, dass Maskenoberteil (3) im Bereich des Nasenrückenbereiches (7) flexibel und weich auszubilden, um einen angenehmen Tragekomfort für den Benutzer zu realisieren. Das Maskenoberteil (3) wird deshalb im Nasenrückenbereich (7) ohne zusätzliche Verwendung des Füllstoffes (15) ausgebildet, oder nur mit einem sehr weichen Füllstoff (15b), mit einer Härte von beispielsweise < 15 Shore 00.

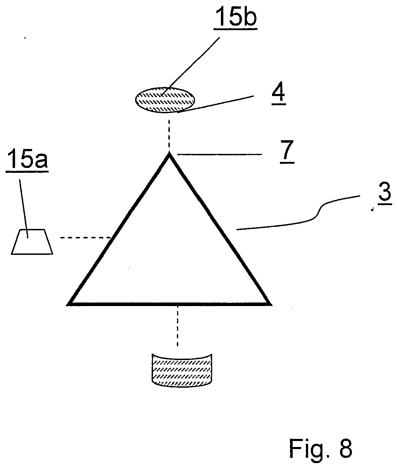

Fig. 8 zeigt einen schematischen Längsschnitt durch das im Wesentlichen dreieckige geformte Maskenoberteil (3) gemäß Fig. 6 und Querschnitte durch den gefüllten Hohlraum (4) an unterschiedlichen Stellen. Ein Querschnitt durch den gefüllten Hohlraum (4) im Nasenrückenbereich (7) zeigt, dass die Füllung (15b) weich und elastisch ausgeführt ist. Der Hohlraum ist im Nasenrückenbereich gerundet in Form einer Ellipse ausgeführt. Ein Querschnitt durch den gefüllten Hohlraum (4) im Seitenbereich zeigt, dass die Füllung (15a) härter ausgeführt ist. Die Form des gefüllten Hohlraumes ist hier (4) eckig, insbesondere trapezförmig, ausgeführt.

Erfindungsgemäß ist die Form des gefüllten Hohlraumes (4) im Verlauf des Umfanges des Maskenoberteils unterschiedlich ausgeführt, so dass sich, bedingt durch die jeweils örtlich verschiedenen Anforderungen an das Maskenoberteil, optimale Dichteigenschaften des Maskenoberteils erzielen lassen.

Die Materialdicke der Wandungen (6) des Maskenoberteils kann in einer weiteren Ausführungsform unterschiedlich gewählt sein, wodurch sie einerseits die nötige Steifigkeit und andererseits einen möglichst weichen und dicht abschließenden Kontakt mit der Haut gewährleistet.

Insbesondere im Bereich der Wandungen (6) des Maskenoberteils, die dem Gesicht anliegen, weist die Wandung eine geringere Materialdicke auf, als die Bereiche der Wandungen des Maskenoberteils, die keinen Gesichtspartien aufliegen.

Bevorzugt sind im Bereich der Wandungen des Maskenoberteils, die dem Nasenrücken (7) anliegen, geringe Materialdicken ausgebildet. Besonders bevorzugt ist die Materialdicke der Wandung in dem Bereich, der dem Nasenrücken anliegt, geringer als in solchen Bereichen der Wandung des Maskenoberteils, die anderen Gesichtspartien aufliegen. Dadurch wird eine optimale Dichtfunktion bei minimalem Druckauftrag im Bereich des empfindlichen Nasenrückens erreicht.

Bevorzugt ist der Hohlraum (4) inhomogen mit Füllstoff (15) ausgefüllt dergestalt, dass gesichtsnahe Bereiche des Füllstoffes weicher und elastischer ausgeführt sind als gesichtsferne Bereiche des Füllstoffes. Dadurch wird zweierlei erzielt, einerseits eine gute Stützfunktion durch den gesichtsfernen härteren Bereich und andererseits eine hohe Dichtfunktion beziehungsweise eine große Flexibilität in dem gesichtsnahen Bereich.

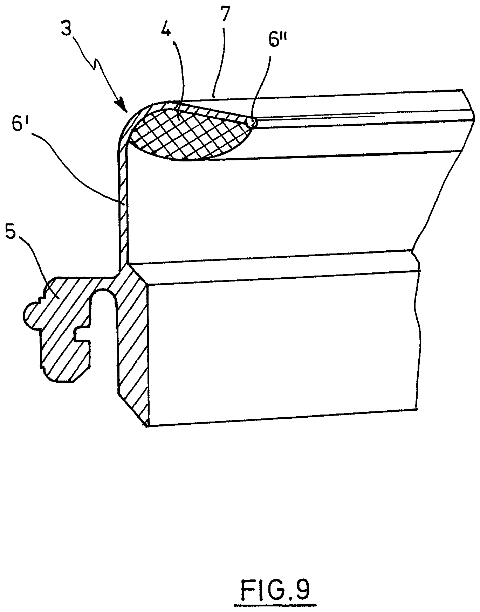

Figur 9 zeigt eine weitere bevorzugte Ausführung der vorliegenden Erfindung wie in Fig. 2 und Fig. 3, bei der das Maskenoberteil (3) zumindest bereichsweise einen begrenzten Hohlraum (4) aufweist. Die Wandung (6') bleibt frei von einem Füllstoff. Als Weiterbildung der Erfindung ist der Wandbereich zudem unterschiedlich stark ausgebildet. Der Wandbereich (6') der frei von einem Füllstoff bleibt, ist dünner und flexibler als die übrige Wandung (6"). Bevorzugt ist das Maskenoberteil im Bereich des Nasenrückens (7) entsprechend Figur 9 ausgebildet. Bedingt durch die dünne Wandung (6') kann sich das Maskenoberteil, insbesondere im Bereich des Nasenrückens, besonders gut der Geometrie des Gesichts anpassen. Bei angelegter Beatmungsmaske und eingeschaltetem Beatmungsgerät herrscht ein Überdruck in der Maske. Der gefüllte Hohlraum liegt dann dichtend an dem Gesicht des Patienten an, während die weiche Wandung (6') durch ihre Flexibilität druckabhängig nach außen gedrückt werden kann, was eine optimale Anpassung an die jeweilige Gesichtskontur unterstützt.

In einer weiteren Ausführungsform der Erfindung ist der gefüllte Hohlraum nur bereichsweise ausgeführt. Bevorzugt befindet sich im Bereich des Nasenrückens kein gefüllter Hohlraum, oder der Hohlraum ist im Nasenrückenbereich nicht gefüllt. In diesem Fall wird eine gute Dichtung und eine hohe Flexibilität des Maskenoberteils (3) im Nasenrückenbereich dadurch erzielt, dass die Wandung (6') hier dünner ist. Bevorzugt ist die Wandung (6') im Nasenrückenbereich maximal dünn ausgeführt.

## Patentansprüche

1. Atemmaske mit einem Maskenoberteil (3), welches aus einem elastischen Material hergestellt ist, wobei das Maskenoberteil (3) durch einen Maskenanschlussbereich (5) mechanisch mit einem Maskenkörper (14) der Atemmaske (8) verbunden ist, und eine Wandung (6) des Maskenoberteiles(3) mindestens bereichsweise einen mindestens bereichsweise mit einem Füllstoff (15) gefüllten Hohlraum (4) begrenzt, welcher zumindest bereichsweise entlang des Umfangs des Maskenoberteils (3) ausgebildet ist, und wobei das Maskenoberteil (3) an seiner dem Maskenkörper (14) abgewandten Seite über eine nach innen geformte, dünne umlaufende Kontaktlippe (1) verfügt, und das Maskenoberteil (3) im Nasenrückenbereich (7) keinen Hohlraum für den Füllstoff aufweist, wobei der Füllstoff (15) ein Gel ist und wobei der Hohlraum (4) derart inhomogen mit Füllstoff (15) ausgefüllt ist, daß gesichtsnahe Bereiche (17) des Füllstoffes (15) weicher und elastischer ausgeführt sind als gesichtsferne Bereich des Füllstoffes (15), wodurch zweierlei erzielt wird, einerseits eine gute Stützfunktion durch den gesichtsferneren härteren Bereich und andererseits eine hohe Dichtfunktion bzw. eine große Flexibilität in dem gesichtsnahen Bereich, **dadurch gekennzeichnet, dass** der Füllstoff (15) zumindest bereichsweise eine Härte zwischen etwa 10 Shore 00 und etwa 20 Shore 00 aufweist.

2. Atemmaske nach Anspruch 1, **dadurch gekennzeichnet, dass** die Dicke der Wandung (6) sich an zumindest zwei Orten entlang des Umfangs des Maskenoberteils (3) unterscheidet.

3. Atemmaske nach einem der Ansprüche 1 oder 2, **dadurch gekennzeichnet, dass** die Eigenschaften des Füllstoffes (15) im Verlauf des Umfanges des Maskenoberteiles (3) zumindest bereichsweise inhomogen sind.

4. Atemmaske nach einem der Ansprüche 1 bis 3, **dadurch gekennzeichnet, dass** die Eigenschaften der Wandung (6) im Verlauf eines Querschnitts des Maskenoberteiles (3) zumindest bereichsweise inhomogen sind.

5. Atemmaske nach einem der Ansprüche 1 bis 4, **dadurch gekennzeichnet, dass** der Füllstoff (15) im Verlauf eines Querschnitts des Maskenoberteiles (3) verschiedene Viskositäts-, bzw. Härtegrade aufweist.

6. Atemmaske nach einem der Ansprüche 1 bis 5, **dadurch gekennzeichnet, daß** das Maskenoberteil (3) im Nasenrückenbereich (7) keinen Hohlraum für den Füllstoff aufweist.

7. Atemmaske nach einem der Ansprüche 1 bis 5, **dadurch gekennzeichnet, dass** das Maskenoberteil (3) im Nasenrückenbereich (7) einen mit einem Füllstoff gefüllten Hohlraum (4) aufweist und daß der Querschnitt des Hohlraums (4) im Nasenrückenbereich (7) minimal ist.

8. Atemmaske nach einem der vorhergehenden Ansprüche, **dadurch gekennzeichnet, dass** das Maskenoberteil (3) im Nasenrückenbereich (7) eine Wandung (6') mit minimaler Dicke aufweist.

9. Atemmaske (3) mit einem Füllstoff (15) nach einem der vorhergehenden Ansprüche, **dadurch gekennzeichnet, dass** im Bereich des Nasenrückens auf eine Unterlegung der lippennahen Bereiche des Maskenoberteils (3) mit einem Füllstoff verzichtet wird und/oder der zu befüllende Hohlraum sehr dünn gestaltet wird.

10. Verfahren zur Herstellung eines Maskenoberteils einer Atemmaske, gemäß Anspruch 1 bei dem das Maskenoberteil (3) mindestens bereichsweise mit einem Hohlraum (4) aus einem flexiblen Material geformt, und mit mindestens einer Öffnung zum Einfüllen des Füllstoffs (15) versehen wird, in welche der Füllstoff (15) eingefüllt wird, **dadurch gekennzeichnet, dass** die Viskosität, bzw. der Härtegrad des einzufüllenden Füllstoffs mit dem Befüllungsstand des Hohlraums variiert wird, wobei als Füllstoff (15) ein Gel verwendet wird und wobei das Maskenoberteil (3) mindestens bereichsweise den Hohlraum (4) begrenzt und wobei das Maskenoberteil (3) an seiner einem Maskenkörper (14) abgewandten Seite über eine nach innen geformte dünne, umlaufende Kontaktlippe verfügt, wobei das Maskenoberteil (3) im Nasenrückenbereich (7) keinen Hohlraum aufweist und wobei der Füllstoff (15) zumindest bereichsweise eine Härte zwischen etwa 10 Shore 00 und etwa 20 Shore 00 aufweist.

11. Verfahren zur Herstellung eines Maskenoberteils einer Atemmaske, gemäß Anspruch 1 **dadurch gekennzeichnet, dass** eine geformte Wulst auf eine Wandung (6) des Maskenoberteils (3) aus einer Düse aufgespritzt wird, wobei die Füllstoffmenge mit einer Härte zwischen etwa 10 Shore 00 und etwa 20 Shore 00 variiert aufgetragen wird und hierdurch eine gewünschte Formgebung erreicht wird, wobei das Maskenoberteil (3) im Nasenrückenbereich (7) keinen Hohlraum aufweist

12. Verfahren nach Anspruch 10 oder 11, **dadurch gekennzeichnet, dass** die auf die Wandung (6) aufgespritzte Wulst mit einer schützenden Schicht versehen wird.

13. Verwendung eines Füllstoffs (15) in einem Maskenoberteil (3) einer Atemmaske (8) gemäß Anspruch 1 und in einem Verfahren zur Herstellung des Maskenoberteils (3), gemäß Ansprüche 10 oder 11 zum Vorgeben von einer im Verlauf ihres Querschnitts variierenden Elastizität der Wandungen (6) des Maskenoberteils (3), wobei das Maskenoberteil (3) mindestens bereichsweise den Hohlraum (4) begrenzt und wobei das Maskenoberteil (3) an seiner einem Maskenkörper (14) abgewandten Seite über eine nach innen geformte dünne, umlaufende Kontaktlippe verfügt, wobei das Maskenoberteil (3) im Nasenrückenbereich (7) keinen Hohlraum aufweist und wobei der Füllstoff (15) zumindest bereichsweise eine Härte zwischen etwa 10 Shore 00 und etwa 20 Shore 00 aufweist.

## Claims

1. Respiratory mask with an upper mask part (3) which is produced from an elastic material, wherein the upper mask part (3) is connected mechanically to a mask body (14) of the respiratory mask (8) by a mask attachment region (5), and a wall (6) of the upper mask part (3) delimits at least in part a cavity (4) which is filled at least in part with a filling material (15) and which is formed at least in part along the circumference of the upper mask part (3), and wherein the upper mask part (3), at the side thereof directed away from the mask body (14), has an inwardly formed, thin peripheral contact lip (1), and the upper mask part (3) has no cavity for the filling material in the nose bridge region (7), wherein the filling material (15) is a gel, and wherein the cavity (4) is filled non-homogeneously with filling material (15), in such a way that regions (17) of the filling material (15) that are near the face are softer and more elastic than regions of the filling material (15) that are remote from the face, the results of which are twofold, on the one hand a good support function afforded by the harder region more remote from the face, and on the other hand a high sealing function or a great flexibility in the region near the face, **characterized in that** the filling material (15) has at least in part a hardness of between approximately 10 Shore 00 and approximately 20 Shore 00.

2. Respiratory mask according to Claim 1, **characterized in that** the thickness of the wall (6) differs at at least two locations along the circumference of the upper mask part (3).

3. Respiratory mask according to either of Claims 1 and 2, **characterized in that** the properties of the filling material (15) are at least in part non-homogeneous along the course of the circumference of the upper mask part (3).

4. Respiratory mask according to one of Claims 1 to 3, **characterized in that** the properties of the wall (6) are at least in part non-homogeneous along the course of a cross section of the upper mask part (3) .

5. Respiratory mask according to one of Claims 1 to 4, **characterized in that** the filling material (15) has different degrees of viscosity or hardness along the course of a cross section of the upper mask part (3) .

6. Respiratory mask according to one of Claims 1 to 5, **characterized in that** the upper mask part (3) has no cavity for the filling material in the nose bridge region (7).

7. Respiratory mask according to one of Claims 1 to 5, **characterized in that** the upper mask part (3) has, in the nose bridge region (7), a cavity (4) filled with a filling material, and **in that** the cross section of the cavity (4) in the nose bridge region (7) is minimal.

8. Respiratory mask according to one of the preceding claims, **characterized in that** the upper mask part (3) has, in the nose bridge region (7), a wall (6') with minimal thickness.

9. Respiratory mask (3) with a filling material (15) according to one of the preceding claims, **characterized in that**, in the region of the nose bridge, the regions of the upper mask part (3) near the lips are not lined with a filling material, and/or the cavity to be filled is very thin.

10. Method for producing an upper mask part of a respiratory mask according to Claim 1, in which method the upper mask part (3) is formed at least in part with a cavity (4) from a flexible material and is provided with at least one opening for the introduction of the filling material (15), into which opening the filling material (15) is introduced, **characterized in that** the viscosity or the degree of hardness of the filling material to be introduced varies with the filling level of the cavity, wherein a gel is used as filling material (15), and wherein the upper mask part (3) at least in part delimits the cavity (4), and wherein the upper mask part (3), at the side thereof directed away from the mask body (14), has an inwardly formed, thin peripheral contact lip, wherein the upper mask part (3) has no cavity in the nose bridge region (7), and wherein the filling material (15) has at least in part a hardness of between approximately 10 Shore 00 and approximately 20 Shore 00.

11. Method for producing an upper mask part of a respiratory mask according to Claim 1, **characterized in that** a shaped bead is injected onto a wall (6) of the upper mask part (3) from a nozzle, wherein the amount of filling material applied has a hardness varying between approximately 10 Shore 00 and approximately 20 Shore 00, and in this way a designed shaping is achieved, wherein the upper mask part (3) has no cavity in the nose bridge region (7) .

12. Method according to Claim 10 or 11, **characterized in that** the bead injected onto the wall (6) is provided with a protective layer.

13. Use of a filling material (15) in an upper mask part (3) of a respiratory mask (8) according to Claim 1 and in a method for producing the upper mask part (3) according to Claim 10 or 11, for predefining an elasticity of the walls (6) of the upper mask part (3), which elasticity varies along the course of its cross section, wherein the upper mask part (3) at least in part delimits the cavity (4), and wherein the upper mask part (3), at the side thereof directed away from the mask body (14), has an inwardly formed, thin peripheral contact lip, wherein the upper mask part (3) has no cavity in the nose bridge region (7), and wherein the filling material (15) has at least in part a hardness of between approximately 10 Shore 00 and approximately 20 Shore 00.

## Revendications

1. Masque respiratoire comprenant une partie supérieure de masque (3) qui est fabriquée à partir d'un matériau élastique, la partie supérieure de masque (3) étant connectée mécaniquement par une région de raccordement de masque (5) à un corps de masque (14) du masque respiratoire (8), et une paroi (6) de la partie supérieure de masque (3) délimitant au moins en partie une cavité (4) remplie au moins en partie avec une charge (15) qui est réalisée au moins en partie le long de la périphérie de la partie supérieure de masque (3), et la partie supérieure de masque (3) disposant, au niveau de son côté opposé au corps de masque (14), d'une lèvre de contact (1) périphérique mince formée vers l'intérieur et la partie supérieure de masque (3) ne présentant pas de cavité pour la charge dans la région de la base du nez (7), la charge (15) étant un gel et la cavité (4) étant remplie avec la charge (15) de manière inhomogène de façon à ce que des régions (17) de la charge (15) proches du visage soient réalisées de manière plus molle et plus élastique que la région de la charge (15) éloignée du visage, de sorte que l'on obtienne à la fois d'une part une bonne fonction de support par la région plus dure éloignée du visage et d'autre part une fonction d'étanchéité élevée ou une grande flexibilité dans la région proche du visage, **caractérisé en ce que** la charge (15) présente au moins en partie une dureté comprise entre environ 10 Shore 00 et environ 20 Shore 00.

2. Masque respiratoire selon la revendication 1, **caractérisé en ce que** l'épaisseur de la paroi (6) est différente au niveau d'au moins deux endroits le long de la périphérie de la partie supérieure de masque (3).

3. Masque respiratoire selon l'une des revendications 1 et 2, **caractérisé en ce que** les propriétés de la charge (15) sont, au moins en partie, inhomogènes le long de la périphérie de la partie supérieure de masque (3).

4. Masque respiratoire selon l'une quelconque des revendications 1 à 3, **caractérisé en ce que** les propriétés de la paroi (6) sont, au moins en partie, inhomogènes le long d'une section transversale de la partie supérieure de masque (3) .

5. Masque respiratoire selon l'une quelconque des revendications 1 à 4, **caractérisé en ce que** la charge (15), le long d'une section transversale de la partie supérieure de masque (3), présente des degrés de viscosité ou de dureté différents.

6. Masque respiratoire selon l'une quelconque des revendications 1 à 5, **caractérisé en ce que** la partie supérieure de masque (3) ne présente pas de cavité pour la charge dans la région de la base du nez (7).

7. Masque respiratoire selon l'une quelconque des revendications 1 à 5, **caractérisé en ce que** la partie supérieure de masque (3) présente, dans la région de la base du nez (7), une cavité (4) remplie de charge et **en ce que** la section transversale de la cavité (4) est minimale dans la région de la base du nez (7).

8. Masque respiratoire selon l'une quelconque des revendications précédentes, **caractérisé en ce que** la partie supérieure de masque (3) présente, dans la région de la base du nez (7), une paroi (6') d'épaisseur minimale.

9. Masque respiratoire (3) comprenant une charge (15) selon l'une quelconque des revendications précédentes, **caractérisé en ce que** dans la région de la base du nez, on omet de garnir les régions proches des lèvres de la partie supérieure du masque (3) avec une charge et/ou la cavité à remplir est configurée sous forme très mince.

10. Procédé de fabrication d'une partie supérieure de masque d'un masque respiratoire selon la revendication 1, dans lequel la partie supérieure de masque (3) est formée au moins en partie avec une cavité (4) constituée d'un matériau flexible, et est pourvue d'au moins une ouverture pour l'introduction de la charge (15) dans laquelle la charge (15) est introduite, **caractérisé en ce que** la viscosité, ou le degré de dureté, de la charge à introduire est modifié(e) en fonction du degré de remplissage de la cavité, un gel étant utilisé en tant que charge (15) et la partie supérieure de masque (3) délimitant au moins en partie la cavité (4) et la partie supérieure de masque (3) disposant, au niveau de son côté opposé à un corps de masque (14), d'une lèvre de contact périphérique mince formée vers l'intérieur, la partie supérieure de masque (3) ne présentant pas de cavité dans la région de la base du nez (7) et la charge (15) présentant au moins en partie une dureté comprise entre environ 10 Shore 00 et environ 20 Shore 00.

11. Procédé de fabrication d'une partie supérieure de masque d'un masque respiratoire selon la revendication 1, **caractérisé en ce qu'**un bourrelet façonné est moulé par injection sur une paroi (6) de la partie supérieure de masque (3) à partir d'une buse, la quantité de charge étant appliquée de manière variable avec une dureté comprise entre environ 10 Shore 00 et environ 20 Shore 00 et de ce fait on obtient une forme souhaitée, la partie supérieure de masque (3) ne présentant pas de cavité dans la région de la base du nez (7).

12. Procédé selon la revendication 10 ou 11, **caractérisé en ce que** le bourrelet moulé par injection sur la paroi (6) est pourvu d'une couche de protection.

13. Utilisation d'une charge (15) dans une partie supérieure de masque (3) d'un masque respiratoire (8) selon la revendication 1 et dans un procédé de fabrication de la partie supérieure de masque (3) selon la revendication 10 ou 11, pour prédéfinir une élasticité des parois (6) de la partie supérieure de masque (3), variant le long de sa section transversale, la partie supérieure de masque (3) délimitant au moins en partie la cavité (4) et la partie supérieure de masque (3) disposant, au niveau de son côté opposé à un corps de masque (14), d'une lèvre de contact périphérique mince formée vers l'intérieur, la partie supérieure de masque (3) ne présentant pas de cavité dans la région de la base du nez (7) et la charge (15) présentant au moins en partie une dureté comprise entre environ 10 Shore 00 et environ 20 Shore 00.
